# EUROPÄISCHE PATENTANMELDUNG

(11) **EP 2 633 765 A1**
(43) Veröffentlichungstag der Anmeldung: **04.09.2013**
(21) Anmeldenummer: 13000887.3
(22) Anmeldetag: 22.02.2013
(51) Int. Cl.: A23L 2/39, A23L 2/52, A61K 9/00

(54) **Instant-Zubereitungen mit Polydextrose, Herstellung und Verwendung**

(30) Priorität: 01.03.2012 DE 102012004307; 01.03.2012 DE 202012006723 U
(71) Anmelder: Merz Pharma GmbH & Co. KGaA, 60318 Frankfurt am Main (DE)
(72) Erfinder: Hövelmann, Julia, 60322 Frankfurt/Main (DE); Hönkemeyer, Nadine, 60486 Frankfurt/Main (DE); Berlekamp, Uwe, 31860 Emmertal (DE)
(74) Vertreter: Müller, Claudia

(57) **Zusammenfassung**

Die vorliegende Erfindung betrifft eine Instant-Zubereitung zur Herstellung eines flüssigen Getränks wie Tee, Vitamindrink, Extrakt-Getränk, durch Aufbereiten mit einer vorzugsweisen heißen Flüssigkeit, insbesondere Wasser. Die Instant-Getränkezubereitung ist pulvrig und ist hergestellt auf Basis von Polydextrose. Sie ist insbesondere im Wesentlichen zuckerfrei und damit kalorienarm, verfügt über ein ausgezeichnetes Auflösungsvermögen und eine besonders hohe Verträglichkeit, wobei die Instantprodukte lagerstabil bleiben.

## Beschreibung

### Gegenstand der Erfindung

Die vorliegende Erfindung betrifft eine Instant-Zubereitung zur Herstellung eines flüssigen Getränks wie Tee, Vitamin- oder Mineralstoffdrink, Pflanzen-Extrakt-Getränk, oder eine Mischung daraus durch Aufbereiten mit einer vorzugsweisen heißen Flüssigkeit, insbesondere Wasser. Die Instant-Getränkezubereitung ist pulvrig hergestellt auf Basis von Polydextrose. Sie ist insbesondere im Wesentlichen zuckerfrei und damit kalorienarm, lagerstabil, dennoch geschmacksintensiv und verfügt über ein gutes Auflösungsvermögen und eine besonders hohe Verträglichkeit.

### Stand der Technik

Unter dem Begriff "Instant-Produkte" werden im Allgemeinen feste, pulverförmige, in einer vorzugsweise heißen Flüssigkeit wie Wasser lösliche oder dispergierbare Produkte als Genussmittel, auch mit Heilwirkungen, mit den jeweiligen Wirkstoffen verstanden. Hierzu gehören z. B. Kaffee, Tee, Frucht- oder Mischgetränke. Dabei werden die Wirkstoffe oder Wirkstoffkombinationen zunächst auf bekannte Weise hergestellt, entweder durch Mischung von Pulvern oder Pulverextrakten oder Herstellung/Extraktion flüssiger Mischungen mit anschließender Trocknung oder ggf. Gefriertrocknung. Es werden dadurch Konzentrate der Ausgangssubstanzen erhalten. Beispiele für solche Ausgangsprodukte sind Extrakte bzw. Extraktgemische aus teeähnlichen Erzeugnissen, wie Hibiskus, Hagebutten, Holunderbeeren, Orangen- und Zitronenschalen, Fenchel, Anis, Süßholz, Ingwer und sonstige Kräuter- bzw. Fruchtextrakte, die als geschmacksgebende oder wirkungsgebende Rohstoffe zur Herstellung des Instant-Pulvers, -granulats oder -konzentrats dienen können. Diese werden anschließend mit einem geeigneten festen Träger vermischt, um das Instant- Getränk zu erhalten. Der Träger ist dabei so auszuwählen, dass einerseits eine gute Verarbeitbarkeit/Dosierbarkeit und andererseits die geschmackliche Komponente des Wirkstoff(kombinations)konzentrats erreicht werden.

Die erhaltenen Pulver sollten darüber hinaus auch eine geeignete Korngröße aufweisen, damit die Portionierung und Verpackung problemlos erfolgen kann. Gegebenenfalls können zu kleine Korngrößen zu einer hohen Staubentwicklung führen, was sowohl bei ihrer Herstellung als auch bei ihrer Verpackung und schließlich auch bei der Handhabung durch den Endverbraucher nachteilig ist. Auch kann dies zu Verklumpungen und damit zu einer schlechteren Löslichkeit/Produktqualität führen.

Zur Lösung dieses Problems wird entsprechend der DE 202005013596 U1 (siehe auch DE 10 2005 040 666 A1) vorgeschlagen, für Instant- Zusammensetzungen Agglomerate auf Basis mindestens eines Agglomeratbildners, welcher aus der Gruppe von Zuckeraustauschstoffen, Stärkederivaten, Dextrose (D-Glucose), Lactose und Maltose ausgewählt ist, bereitzustellen. Dabei sollen die Zusammensetzung bzw. die Agglomerate eine Schüttdichte von 100 bis 300 g/l, vorzugsweise 125 bis 275 g/l, insbesondere 150 bis 250 g/l aufweisen, während bis dahin bekannte Instant- Produkte deutlich höhere Schüttdichten von üblicherweise 400 bis 500 g/l zeigten.

Zu allgemeinen Herstellungsverfahren von Instant-Produkten wird hier auf Römpp Chemielexikon, Band 3, 10. Auflage, Georg-Thieme-Verlag, Stuttgart/New York, 1997, Seite 1936, Stichwort: "Instant-Produkte", sowie die dort referierte Literatur hingewiesen.

Die Veröffentlichung DE 202008007759 U1 adressiert das o.g. Problem der Korngröße durch Instant-Getränkezusammensetzungen, welche 1 bis 99 Gew.% (vor allem 30-75 Gew.%) eines Granulatbildners und mindestens einen Geschmacksbildner aufweisen. Erstere sind ausgewählt aus Zuckeralkoholen, Süßstoffen, Stärkederivaten, Zuckern. Dazu gehören vor allem Erythrit, Mannit, Xylit, Sorbit, Isomaltit, Maltit, Lactit, Galactit, Acesulfam, Cyclamat, Glycyrrhizin, Aspartam, Dulcin, Saccharin, u.ä., Saccharose, Mannose, Maltose, Dextrose, Lactose, Glucose, Xylose, Isomaltulose, ferner aus Stärkeabbauprodukten wie Dextrine (Maltodextrine). Als Geschmacksbildner kommen insbesondere Pflanzenextrakte, Fruchtextrakte, Tee-Extrakte infrage.

Aus der DE 202010001912 U1 sowie der DE 102010009654 A1 sind analoge Instant- Zubereitungen bekannt, welche als Granulat vorliegen und mindestens einen Granulatbildner (1-99 Gew.%) sowie mindestens einen Pflanzenbestandteil umfassen. Dabei ist der Granulatbildner ausgewählt aus der Gruppe von Zuckern, Süßstoffen, Zuckeralkoholen, Stärkederivaten, entsprechend den zuvor oben genannten Substanzen, insbesondere Saccharose (Kristallzucker), Mannose, Maltose, Glucose (Dextrose), Lactose, Xylose, Isomaltulose.

Aus der DE 41 23 124 A1/C1 sind Instant Getränkemischungen bekannt, welche als granulatförmigen oder pastösen Träger Inulin (Fructosepolysaccharid mit endständigem Glucoserest) aufweisen. Damit kann das Problem der kariogenen Wirkung der üblichen Zuckerträgerstoffe wie genannt (vor allem Zucker (Saccharose), Glucose (Dextrose, Traubenzucker), Glucosesirupe oder Maltodextrine umgangen werden.

Die WO 2006/108592 A1 betrifft ein Verfahren zur Herstellung von festen, geformten Instantstücken z.B. in Form eines Würfels (Brühwürfel) durch Vakuumtrocknen einer wässrigen Instantzusammensetzung. Durch dieses Trocknen soll die Instantzubereitung eine bessere Matrix und damit eine verbesserte Löslichkeit erhalten. Der Trägerstoff besteht aus Zucker oder zuckerähnlichem Werkstoff, bevorzugt Isomaltulose oder Inulin. Als strukturgebender Trägerstoff ist allgemein Polydextrose genannt.

Die WO 2010/020321 beschreibt ein Polydextrose-Material mit einem Polymerisationsgrad von 5 und mehr. Dieses Material kann als Zusatz in pharmazeutischen Produkten oder in Lebensmitteln, insbesondere als Befeuchtungsmittel, in Mengen von bis zu 19 Gew.% z. B. in Schokolade mit ca. 45 Gew.% Fett oder Fett und Milchpulver als Träger gewählt werden.

Die WO 00/11971 A2 offenbart eine feste Getränkezubereitung zur Herstellung flüssiger Getränke in Scherformmatrix als Folge eines Schnellschmelzverfahrens (flash flow processing). Diese Technik soll zu einer größeren Oberfläche und damit verbesserten Löslichkeit ohne Verklumpen des Produktes (in Wasser) führen. Die trockene Getränkezubereitung weist ferner insbesondere essbare Faserstoffe (Ballaststoffe) neben Sacchariden wie vor allem Maltodextrin, ggf. Polydextrose ohne weitere Charakterisierung auf.

Die WO 2011/141150 A1 betrifft eine Kakaopulverzubereitung mit 70 bis 99,5 % Kakaopulver und 0,2 bis 20 % eines hydrophilen Mittels, wobei das Kakaopulver mit dem hydrophilen Mittel überzogen (gecoated) ist. Das Kakaopulver kann mit einem Zucker wie Maltose, Sucrose, oder Polydextrose gemischt sein.

Der Stand der Technik schlägt demnach zur Herstellung von Instant- Produkten den Einsatz vor allem herkömmlich bekannter Zucker und/oder Zuckerersatzstoffe vor, um sowohl eine entsprechende galenische Handhabbarkeit (Korngröße) als auch eine geeignete Geschmacksintensität zu erzielen. Übliche Zucker sind aber bekanntermaßen für den Organismus in höheren Mengen schädlich (Zähne, Gewicht). Andererseits weist Inulin für die Anwender unangenehmen Nebenwirklungen (Flatulationen) auf.

### Aufgabe vorliegender Erfindung

Aufgabe vorliegender Erfindung ist, eine Instant-Getränkezubereitung bereitzustellen, welche sich zur Herstellung eines vorzugsweise kalorienreduzierten Instant-Getränkes durch Aufbereiten mit einer Trinkflüssigkeit eignet, wobei die zuvor geschilderten Nachteile des Standes der Technik zumindest im Wesentlichen vermieden oder aber zumindest verringert werden können, ohne dass es zu Qualitäts- oder Lagerproblemen kommt.

### Lösung der Aufgabe

Diese Aufgabe wird erfindungsgemäß gelöst durch Instant- Zubereitungen, welche eine Wirkstoffkomponente aufweisen sowie als Trägerstoff Polydextrose enthalten. Derartige im Wesentlichen feste, pulverförmige Zubereitungen eignen sich zur Herstellung eines Getränkes, indem eine trinkbare Flüssigkeit wie vor allem Wasser, insbesondere heißes Wasser, hinzugegeben wird. Die Zubereitung ist leicht löslich und weist eine jeweils verbraucherbezogene, insbesondere kalorienarme Süßung (Zuckerersatzstoffe) auf. Die Schüttdichte (Masse/-Volumen) liegt im Bereich von 400 - 900 g/l (abhängig von der Korngrößenverteilung) und entspricht damit herkömmlichen Instant - Produkten (400-500 g/I). Vorzugsweise weisen die anmeldungsgemäßen Instant- Zubereitungen mehr als 50 Gew. %, vor allem mehr als 56 Gew.% Polydextrose (Trägerstoff) auf. Bevorzugt ist Polydextrose mit einer mittleren molaren Masse von 100 bis 25000 g/Mol, vor allem 150 bis 10000 g/ Mol, insbesondere 180 bis 5000 g/Mol.

Das anmeldungsgemäße Instant- Produkt bleibt lagerbeständig, ohne dass eine Feuchtung bzw. ein Verklumpen die Qualität vermindern würde. Darüber hinaus ist mit dem anmeldungsgemäßen Produkt eine ausreichende, insbesondere hohe Geschmacksintensität erreichbar, auch ohne dass eine wie bisher übliche Menge an Geschmacksträgern, insbesondere Zuckern, oder andere Süßungsmittel wie z. B. 50 und mehr Gew.%, erforderlich wäre. Dies ist auch deshalb überraschend, weil Polydextrose zwar als Teilfüllstoff bzw. Zucker- und Fettersatzstoff für Lebensmittel bekannt war, wobei dort aber auch dessen feuchthaltende Eigenschaft bekannt und ggf. gewünscht war und ein vollständiger Ersatz als Geschmacksträger - wegen der normalerweise fehlenden Süße - nicht in Betracht gezogen wurde.

Die erfindungsgemäße feste Instant- Zubereitung umfasst insofern einen Trägerstoff, ein oder mehrere Wirkstoffe (Wirkstoffkomponente), ausgewählt aus Pflanzenextrakten, Pflanzenpulvern, Fruchtkonzentraten, Fruchtextrakten, Vitaminen, Mineralstoffen, Aminosäuren oder Kombinationen hiervon, weiterhin Zusatzstoffe, ausgewählt aus Geschmacks-/Aromastoffen, Säureregulatoren, Füllstoffen, Farbstoffen, Süßungsmitteln, Trennmitteln, oder Mischungen hiervon, wobei als Trägerstoff Polydextrose enthalten ist.

Vorzugsweise umfasst die Zubereitung 65 bis 95 Gew.%, vor allem 75-92 Gew.% der Polydextrose, sowie weiterhin 0,1 bis 18 Gew.% einer oder mehrerer Wirkstoffkomponenten und als Rest übliche Zusatzstoffe , ausgewählt aus Aromen/Geschmacksstoffen, Säureregulatoren, Füllstoffen (insbesondere Aerosil), Farbstoffen, (vor allem niedrig kalorischen) Süßungsmitteln, und/oder Trennmitteln.

Insbesondere ist die Zubereitung im Wesentlichen frei (z.B. weniger als 0,5 g Zucker auf 100 g oder 100 ml) von Zuckern wie vor allem Glucose, Maltose, Saccharose (Kristallzucker), Mannose, Lactose, Xylose, Isomaltulose, oder Mischungen hiervon oder im Wesentlichen frei von Inulin oder auch frei sowohl von den genannten Zuckern oder Mischungen hiervon als auch frei von Inulin.

Ganz besonders bevorzugt sind die Zubereitungen frei von Inulin. Auf den Einsatz von Fetten kann im Allgemeinen verzichtet werden (weniger als 0,5 Gew. %), außer es handelt sich um technologisch notwendige Zusatzstoffe, wie Trennmittel, z.B. Speisefettsäuren oder deren Salze oder pflanzliches Öl wie Sonnenblumenöl, welche als Trennmittel verwendet werden können.

Bevorzugt sind auch im Wesentlichen keine höher kalorischen Zuckerausstoffstoffe wie vor allem Zuckeralkohole aus der Gruppe umfassend Erythrit (Erythritol), Mannit (Mannitol), Xylit (Xylitol), Sorbit (Sorbitol), Isomaltit bzw. Isomalt, Maltit (Maltitol), Lactit (Lactitol), Galactit (Galactitol) sowie deren Mischungen enthalten.

Alle Mengenangaben sind Gewichtsprozent, bezogen auf die Gesamtzubereitung, sofern nicht anders angegeben.

### Nähere Erläuterung der Zubereitung/Inhaltsstoffe

Die Inhaltsstoffe der erfindungsgemäßen Instant- Zubereitung sind im Allgemeinen natürlich oder naturidentisch und damit lebensmittelzulässig und unbedenklich.

### a) Wirkstoffe (Wirkstoffkomponente)

Als Wirkstoffe (Wirkstoffkomponente) kommen vorzugsweise Vitamine wie Vitamin C, B-Vitamine, Mineralien wie vor allem Calcium, Magnesium, Zink in Form entsprechender üblicher Salze wie Chlorid, Phosphat, Carbonat, Hydrogencarbonat, Gluconat, Hydroxid, Nitrat, Citrat infrage. Besonders geeignet sind Calciumcarbonat, Zinkgluconat, Magnesiumhydrogencarbonat, einzeln oder in Kombination.

Auch Aminosäuren wie Cystein (oder Cystin), Histidin, Arginin, können in erfindungsgemäßen Zubereitungen enthalten sein.

Weiterhin geeignet sind Extrakte, ggf. Öle und Pulver aus Pflanzen, Früchten, Säften wie Pflanzen-, Fruchtextrakte und/oder Tee-Extrakte oder -Pulver wie Melissenextrakt, Minzeextrakt, Zimtpulver, (Pfeffer)minzextrakt, Eukalyptusextrakt, Traubensaftkonzentrat bzw. Extrakte hiervon, Hibiskusextrakt, Ananasextrakt, Mangoextrakt, Mate, Ingwerwurzelextrakt -pulver, Anis-, Fenchel, Lindenblüten-, Süßholzwurzelextrakt, Kakaobohnenextrakt, Zitronensaftkonzentrat, Orangensaftkonzentrat, Zitronenfruchtpulver, Hagebuttenextrakt, Acerolaextrakt, Bärentraubenextrakt, Weißdornfrüchtepulver, Ginkgoextrakt, Ginsengextrakt, Pelargonium-Extrakt, Zitronengras, Guarana, Granatapfel, Cranberry, Birkenextrakt oder Mischungen derartiger Wirkstoffe.

Die Extrakte, Konzentrate, Pulver sind bekannt bzw. werden nach bekannten Methoden hergestellt und sind im Handel erhältlich. Bevorzugt werden Trockenprodukte eingesetzt. Die Pflanzen-Extrakte können z.B. durch Lösungsmittelextraktion (mit Alkoholen, Triglyceriden oder Kohlenwasserstoffen, Wasser, Gemischen hiervon) erhalten und als solche oder ggf. nach Trocknung (Sprühtrocknung, Mahlen) dann eingesetzt werden.

Extrakt-Öle werden auf bekannte Weise wie durch Wasserdampfdestillation hergestellt. Hierdurch werden z.B. ätherische Öle aus den genannten Pflanzen erhalten, welche ebenfalls und insbesondere als Aromastoffe eingesetzt werden können.

Vorzugsweise sind als Wirkstoffe Vitamine wie Vitamin C, Mineralien, Aminosäuren und /oder Pflanzenextrakte, Fruchtextrakte, Pflanzenpulver, Fruchtpulver oder Mischungen hiervon enthalten.

Je nach Wirkstoffhauptkomponente(n) kann daher ein Teegetränk, ein Erfrischungsgetränk, ein Fruchtgetränk, Vitamingetränk, Stärkungsgetränk hergestellt werden. Dazu wird die erfindungsgemäße Instant- Zubereitung vorzugsweise portioniert in einer Menge bereitgestellt, dass mittels der Flüssigkeit ein Getränk für eine Tasse/Glas bereitet werden kann. Die Instant-Zubereitung wird dazu vorzugsweise in einen Portionsbeutel gefüllt, enthaltend 3-20, vorzugsweise 5 bis 10 g Instant-Zubereitung.

### b) Füllstoffe

Als Füllstoffe eignen sich insbesondere Siliciumdioxid (Aerosile), Stärkederivate (Dextrine) wie Maltodextrin und/oder Gummi Arabicum, Cellulosederivate oder Pektine. Diese liegen im Allgemeinen in Mengen von 0,01 bis 5 Gew.% vor.

### c) Säure-Regulatoren und Säuerungsmittel

Als Säure- Regulatoren und Säuerungsmittel kommen vor allem Zitronensäure und Derivate und Salze hiervon wie Monohydrat oder Kaliumcitrat, Natriumcitrat, aber auch Weinsäure, Äpfelsäure bzw. geeignete Derivate der beschriebenen Art infrage.

### d) Süßungsmittel: Zucker/Zuckeraustauchstoffe/künstliche Süßungsmittel.

Falls eine bestimmte Süße in den erfindungsgemäßen Produkten vorliegen soll, können geringere Anteile (weniger als 0,5 g Zucker auf 100 g oder 100 ml) an Zuckern wie Glucose oder Saccharose, oder vorzugsweise auch Honig wie Speisehonig, z.B. Honigpulver eingesetzt werden. Alternativ können auch künstliche Süßungsmittel (ohne hohen kalorischen Brennwert) (z. B. 0,01 bis 5 Gew.%) zu diesem Zweck inkorporiert werden. Dazu gehören vor allem Acesulfam (Beispielsweise auch Acesulfam-K), Cyclamat, Glycyrrhizin, Aspartam, Dulcin, Saccharin (beispielsweise auch Natriumsaccharin und/oder Calciumsaccharin), Rebaudiosid (z. B. Rebaudiosid A), Steviosid, Naringin-Di-hydrochalkon, Monellin, Neohesperidin-Dihydro-chalkon (NHDC), Sucralose, Thaumatin, Neotam sowie deren Mischungen und Kombinationen, insbesondere Aspartam und/oder Acesulfam, bevorzugt Aspartam und Acesulfam.

Es kann aber auch möglich oder ggf. erforderlich sein, dass Zuckerausstoffstoffe (Süßungsmittel mit kalorischem Wert), insbesondere in geringen Mengen wie 0,01 bis 8 Gew. %, eingesetzt werden. Dazu gehören vor allem Zuckeralkohole aus der Gruppe von Erythrit (Erythritol), Mannit (Mannitol), Xylit (Xylitol), Sorbit (Sorbitol), Isomaltit bzw. Isomalt, Maltit (Maltitol), Lactit (Lactitol), Galactit (Galactitol) sowie deren Mischungen und Kombinationen.

Bevorzugt werden als Süßungsmittel Honig, künstliche Süßungsmittel oder Kombinationen hiervon eingesetzt. Insbesondere beträgt der Gesamtanteil hiervon 0,1 bis 8 Gew. %, vorzugsweise 0,1 bis 5 Gew.%, vor allem 0,1 bis 3 Gew.%, bezogen auf die Gesamtzubereitung.

### e) Trennmittel

Als Trennmittel können Speisefettsäuren oder deren Salze oder pflanzliches Öl in jeweils dafür bekannten, geeigneten Mengen verwendet werden.

### f) Farb- und Geschmacksstoffe (Aromen)

Als Farbstoffe kommen natürliche Substanzen wie Riboflavin, vorzugsweise als 5-Phosphat, oder Carotinoide (Beta- oder andere Carotinoide), Lycopine, Luteine, oder auch Pflanzenextrakte wie Rote-Bete-Saftpulver oder natürliche Frucht(saft)zubereitungen oder Extrakte daraus sowie wasserlösliche Farbstoffe, wie z. B. Kurkumin, Riboflavin, Karmin, Indigotin, Chlorophylle und Komplexe daraus oder Zuckerkulöre zum Einsatz.

Als Aroma-(Geschmacks)stoffe eignen sich Pflanzen- und Fruchtaromen wie z.B. Orange, Mango, Zimtaroma, Zitronenaroma, Salbeiaroma, Kirscharoma, Ingweraroma, (Pfeffer)-Minzaroma, Eukalyptusaroma, Traubensaftkonzentrat bzw. Extrakte hiervon. Derartige Produkte, insbesondere ätherische Öle, werden auf bekannte Weise hergestellt (s.o.) und sind im Handel erhältlich.

Die Zubereitung enthält diese Substanzen im Allgemeinen in Mengen von 0,1 bis 5 Gew. %.

### g) Trägerstoff Polydextrose:

Polydextrose ist in der Lebensmittelindustrie als (Teil)Ersatzstoff für Zucker oder Fette bekannt. Sie dient hier als Füllstoff. Bei Polydextrose handelt es sich um unregelmäßig verzweigte Glucosepolymere mit verschiedenen Arten glycosidischer Bindungen, vorrangig 1,6-Bindungen. Hinzu kommen einige Sorbit-Endgruppen sowie Mono- und Diester-Bindungen mit einer Lebensmittelsäure wie Zitronensäure. Die Herstellung von Polydextrose erfolgt durch Polymerisation von Glucose in Anwesenheit von Mineralsäuren oder Carbonsäuren. Das Endprodukt enthält kleine Mengen der Ausgangsprodukte. Die Polydextrose liegt amorph vor und ist gering hygroskopisch, gut hydrolyse-, temperatur- und lagerstabil. Beim Auflösen feingemahlener Polydextrose kann es zu Verklumpungen kommen. Grobkörnige Polydextrose ist hingegen schneller löslich (80 g /100 ml Wasser bei 20°C).

Sie weist einen geringen Kalorienwert (etwa 4,18 kJ/g bzw. 1-2 kcal/g) und ähnliche technologische Eigenschaften wie Saccharose auf. Rohe Polydextrose kann gefärbt oder sauer oder bitter sein (natürlicher pH etwa 2-3). Umgekehrt führen Anteile freier Glucose /Sorbitol zu einer gewissen Süße. Dementsprechend wurden inzwischen verschiedene Verfahren entwickelt, um die Eigenschaften von Polydextrose entsprechend den Erfordernissen einzustellen. Zur Aufreinigung wurde beispielsweise vorgeschlagen, die Polydextrose (Polyglucose, im Allgemeinen meistens erhalten durch Kondensation von Glucose in Anwesenheit von Sorbitol und Zitronensäure) mit Hilfe von Peroxiden zu entfärben und anschließend durch organische Lösungsmittel auszufällen (Entfernung des bitteren Geschmacks), siehe US 4,622,233 B1.

Zahlreiche andere Verfahren sind in der DE 000069418973 T2 offenbart. Dort selbst wird vorgeschlagen, hypokalorige Polydextrose mittels eines Umsetzungsschrittes mit Glucoseoxidase und eines Anionenaustauschers herzustellen. Das so hergestellte Produkt soll sich als Teilersatzstoff (in Kombination mit Maltitol) für Zucker bei der Herstellung von Bonbons oder als Austauschstoff für Fette eignen.

Die WO 2004/075664 A1 betrifft die synergistische Verwendung von vorzugsweise gereinigter Polydextrose und Zuckern zur Geschmacksverstärkung von Lebensmitteln wie Bier, Eis, Desserts, Kaugummi, Sportdrinks.

Eine Eignung oder Verwendung als Trägerstoff in Instant -Getränken ist bisher jedoch nicht beschrieben worden. Die vorliegende Erfindung beschreibt die Verwendung von Polydextrose als Trägerstoff zur Herstellung von festen Instantzubereitungen zur Herstellung von Getränken. Die Instantzubereitungen umfassen weiterhin einen oder mehrere Wirkstoffe, ausgewählt aus Pflanzenextrakten, Pflanzenpulvern, Fruchtkonzentraten, Fruchtextrakten, Aminosäuren, Vitaminen, Mineralstoffen, oder Kombinationen hiervon; Zusatzstoffe, ausgewählt aus Geschmacks-/Aromastoffen, Säureregulatoren, Füllstoffen, Farbstoffen, Süßungsmitteln, Trennmitteln oder Mischungen hiervon. Die verwendete Polydextrose kann insbesondere eine wie oben/ unten beschriebene sein.

Die nach vorstehend beschriebenen Verfahren gewonnenen Polydextrose-Produkte sind für erfindungsgemäße Zubereitungen geeignet. Es handelt sich um weiße bis gelbliche geruchlose Pulver, die als vermahlene Schmelze, klarer Sirup, oder sprühgetrocknetes Produkt im Handel erhältlich sind. Polydextrose ist in Wasser löslich, in organischen Lösungsmitteln wie Alkohol wenig und in Aceton nicht löslich.

Polydextrose ist ein Polykondensationsprodukt aus Glucoseeinheiten der chemischen Formel 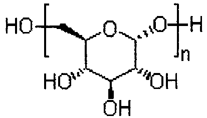 mit einer 1,6-Verknüpfung, die beispielhaft folgende Struktur haben kann (in Abhängigkeit der verknüpften Einheiten):

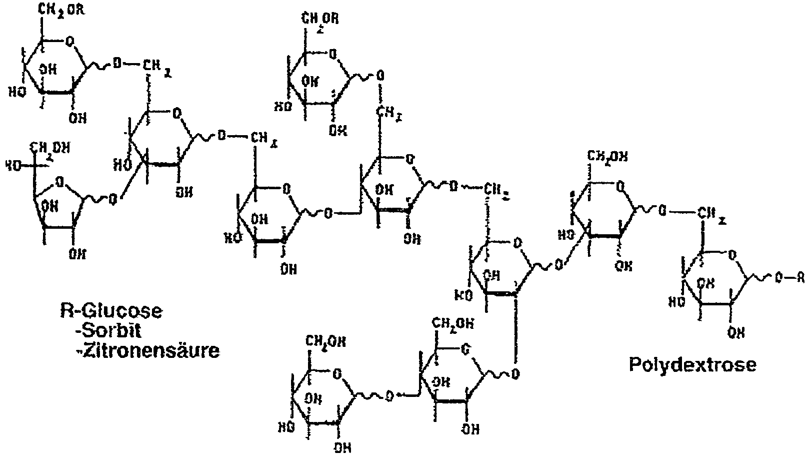

### Beispielhafte Darstellung einer Polydextrose

Bevorzugte Polydextrose kann eine mittlere Molmasse von ca. 100 bis 25000 g/mol, insbesondere 150 bis 10000 g/ mol und bevorzugt 180 bis 5000 g/mol aufweisen. Zu den Polydextrose-Produkten gehören auch hydrogenierte/reduzierte, und fraktionierte Polydextrose-Derivate, die nach bekannten Techniken hergestellt und verkauft werden (siehe auch US 5,601,863, US 5,620,871 und US 5,424,418).

Geeignet ist auch chromatographisch behandelte Polydextrose.

Insbesondere geeignet ist gereinigte Polydextrose, oder auch hydrogenierte/-reduzierte Polydextrose. Besonders geeignet ist gereinigte und hydrogenierte/reduzierte Polydextrose. Die Reinigung erfolgt vorzugsweise mittels Standardtechniken wie Chromatographie, HPLC, wobei es bevorzugt ist, wenn ein Reinheitsgrad von wenigstens 80%, vor allem 85 % vorliegt.

Der pH- Wert der gereinigten Polydextrose liegt im Allgemeinen höher als beim Rohprodukt (etwa 3 und höher), das Produkt ist auch weniger bitter. Die bevorzugt eingesetzte Polydextrose ist daher gereinigte Polydextrose mit einem pH- Wert von 3 und mehr, insbesondere 3,5 bis 6,5 (10 Gew.% in wässriger Lösung).

Derartige Polydextrose-Produkte sind kommerziell erhältlich z. B. von Danisco Sweeteners, unter dem Namen Litesse® Ultra oder Litesse® II, wobei Litesse® Ultra ein reduziertes Produkt bezeichnet. Andere geeignete Polydextrosen der oben beschriebenen Art sind im Handel unter dem Namen Polydextrose A, N von Pfizer oder Winway® Acme® Polydextrose powder bzw. Winway II Polydextrose powder von Shanghai Winway Biotech Co.oder Sta-Lite C90 bzw. R 90 von Tate & Lyle erhältlich.

Polydextrose liegt vor allem in Pulverform vor und kann leicht mit den übrigen erfindungsgemäßen Inhaltsstoffen vermischt werden.

Besonders bevorzugt wird gereinigte Polydextrose mit einem pH Wert von 4,5 bis 6,5 (10 Gew.% in wässriger Lösung) oder gereinigte reduzierte Polydextrose (pH Wert 3,5 bis 5) eingesetzt (Winway II bz. Winway Acme), jeweils Pulver.

Diese gereinigten oder gereinigten reduzierten Polydextrosen können auch eine mittlere molare Masse von 100 bis 5000 g/Mol, insbesondere 180 bis 5000 g/Mol aufweisen.

Polydextrose wird nach dem Verzehr erst im Dickdarm von den dort angesiedelten Bakterien teilweise fermentiert. Etwa die Hälfte der aufgenommenen Poldextrose wird unverändert ausgeschieden. Durch die Fermentation im Dickdarm entstehen hauptsächlich kurzkettige Fettsäuren, die durch Absorption dem Stoffwechsel zugeführt werden. Die Absenkung des pH-Wertes durch die Fermentation im Dickdarm hemmt die Vermehrung pathogener Bakterien. Außerdem wird die Vermehrung von Bifidobakterien und Lactobazillen stimuliert. Es wirkt demnach prebiotisch. Untersuchungen am Menschen zeigen einen kalorischen Brennwert von 1 kcal/g. Dieser Wert wurde zuerst in den USA als physiologischer Brennwert festgelegt und dann von allen anderen Ländern, einschließlich aller EU-Staaten, außer Deutschland übernommen. In Deutschland ist der kalorische Wert auf 2 kcal/g festgelegt. Es wurden die höchsten, bei Tierversuchen, ermittelten Werte zu Grunde gelegt.

Polydextrose kann unbegrenzt eingesetzt werden, da vom Joint FAO/WHO Expert Committee on Food Additives (JEFCA) ein "unspezifischer" ADI erteilt wurde. Der ADI-Wert *(Acceptable Daily Intake)* beziffert die tägliche Aufnahmemenge von Fremdstoffen in Lebensmitteln, die ein Mensch lebenslänglich täglich verzehren kann, ohne gesundheitliche Schäden davonzutragen.

Als Füllstoff gilt für Polydextrose das Prinzip "quantum satis", also unbeschränkter Einsatz. Polydextrose ist physiologisch gut verträglich und kann bedenkenlos in einer Menge von bis zu 90 g/Tag dem Organismus zugeführt werden. Die für Inulin bekannten besonders unangenehmen Nebenwirkungen treten mit Polydextrose nicht auf, da diese unverdaulich und im sauren bis neutralen pH-Wert -Bereich stabil bleibt.

### Bevorzugte Ausführungsformen

Bevorzugte Ausführungsformen anmeldungsgemäßer fester Instant-Zubereitungen umfassen (bevorzugt bestehen aus):
65 bis 95 Gew.%, insbesondere 75 bis 90 Gew.%, Trägerstoff, vor allem ausgewählt aus gereinigter Polydextrose mit einem pH-Wert von 3,5 bis 5 (10%ige wässrige Lösung); gereinigter reduzierter Polydextrose mit einem pH-Wert von 4,5 bis 6,5 (10%ige wässrige Lösung); oder Mischungen hiervon; sowie
1 bis 18 Gew. %, insbesondere 1 bis 8 Gew.% Wirkstoffe, insbesondere ausgewählt aus Melissenextrakt, Zitronenfruchtpulver, Ingwerwurzelpulver, Hagebuttenextrakt, Lindenblütenpulver, Pelargoniumextrakt, Salbeiextrakt, Eukalyptusextrakt, Minzeextrakt, Calciumcarbonat, Zinkgluconat, Magnesiumhydrogencarbonat, Pulvern aus Pflanzen oder Pflanzenteilen wie Wurzeln, Blättern, Blüten, Acerolaextrakt, Bärentraubenextrakt, Weißdornfrüchtepulver, Ginkgoextrakt, Ginsengextrakt, Pelargoniumextrakt, Zitronengras, Guarana, Granatapfel, Cranberry, Birkenextrakt, oder Kombinationen aus einem oder mehreren der genannten Wirkstoffe; und
0,1 bis 20 Gew.% Zusatzstoffe, ausgewählt aus Siliciumdioxid, Zitronensäure, pflanzliche Öle - wie Sonnenblumenöl , Farb- und Geschmacks-/Aromastoffen wie Ingweraroma, Salbeiaroma, Eukalyptusaroma, Zitronenaroma, Orangenaroma, Pfefferminzaroma, Kirscharoma, Acerolaaroma.

Besonders bevorzugter Säureregulator ist Zitronensäure oder ein Hydrat hiervon.

Eine andere Ausführungsform umfasst (bevorzugt besteht aus): anmeldungsgemäße Zubereitungen, worin Polydextrose eingesetzt ist, welche mindestens 90 % Polymer, und max. 0,5 % reduzierten Zucker aufweist, wie z. B. das unter dem Handelsnamen Winway® Acme® Polydextrose Powder von der Firma Shanghai Winway Biotech Co. vertriebene Polydextrose Produkt.

In einer anderen Ausführungsform wird in anmeldungsgemäße Zubereitungen eine gereinigte Polydextrose eingesetzt, welche mindestens 90 % Polymer, max. 4 % Glucose aufweist, wie z. B. das unter dem Handelsnamen Litesse® Two Powder oder Litesse® Ultra Powder von der Firma Danisco vertriebene Polydextrose Produkt.

Weitere derartige Produkte werden im Handel auch unter dem Namen Polydextrose A, N von Pfizer vertrieben.

Bevorzugt weisen derartige Polydextrosen mittlere molare Massen von ca. 180 bis 5000 g/Mol auf.

Geeignet ist auch gereinigte reduzierte Polydextrose mit einem pH-Wert von 4,5 bis 6,5 und einer mittleren molaren Masse von ca. 100 bis 5000 g/Mol, insbesondere 180 bis 5000 g/mol, oder gereinigte Polydextrose mit einem pH- Wert von 3,5 bis 5 und einer mittleren molaren Masse von ca. 100bis 5000 g/Mol, insbesondere 180 bis 5000 g/mol oder Mischungen hiervon.

### Herstellung

Die Herstellung der festen, trockenen Instant-Zubereitungen kann nach folgenden Methoden erfolgen:
Zunächst wird eine Mischung bereitet aus dem Polydextrose Trägermaterial sowie einem oder mehreren Wirkstoffen, ausgewählt aus Pflanzenextrakten/-Pulver-Konzentraten, Mineralstoffen, Vitaminen, Aminosäuren, Proteinen oder Mischungen hieraus. Dazu werden nachfolgend sofern vorhanden die Zusatzstoffe gegeben. Sofern es sich bei der einen oder anderen Komponente um Flüssigkeiten handelt, erfolgt am Ende des Mischungsprozesses eine Trocknung, z. B. bei Temperaturen von 25-130° C. Ebenfalls kann es erforderlich werden, das Mischen in Gegenwart geringer Mengen Lösungsmittel wie vor allem Wasser vorzunehmen. Auch hier erfolgt eine Produkttrocknung am Ende. Die Granulierung erfolgt nach bekannten Maßnahmen wie Sprühgranulation, Pelletisierung, Extrusion, Agglomeration, Naßgranulation, u.ä. in üblichen Vorrichtungen hierfür. Das Mischen bzw. Granulieren kann bei Raumtemperatur oder auch bei Temperaturen im Bereich von 15°C bis 35 °C bei Normaldruck erfolgen.

Es sollte darauf geachtet werden, dass die Gesamtrestfeuchte (umfassend Wasser und/ oder flüssige Substanzanteile) im Instant-Endprodukt etwa 5 Gew% nicht übersteigt und vorzugsweise weniger als 4 Gew.% beträgt.

Je nach Produktspezifität kann im Anschluss an die Granulierung oder Trocknung eine Siebung erfolgen, falls ein Produkt besonders einheitlicher bestimmter Korngröße erwünscht sein sollte.

### Eigenschaften und Anwendungen

Die erhaltenen Instant- Zubereitungen weisen eine gute Dosierbarkeit/Handhabbarkeit mit geringer Staubneigung auf. Sie können daher einfach in Portionierpackungen (Beutel), insbesondere Portioniersachets geeigneter Größe (wie 3-30 g, insbesondere 3-20 g, je nach Anwendungsziel) eingefüllt werden. Die Packungen bzw. Sachets sind vorzugsweise aus einer Papier- oder Aluminiumfolie, einer Verbundfolie oder einer Mischung hieraus hergestellt. Die Erfindung betrifft insofern auch Zubereitungen, die in einer derartigen Portionspackung als Inhalt in einer Menge von 3-30, insbesondere 3-20 g vorhanden sind bzw. Portionspackungen, bestehend aus einem Beutel/Sachet aus Papier- oder Aluminiumfolie, einer Verbundfolie oder einer Mischung hieraus und der angegebenen Menge an der beschriebenen erfindungsgemäßen Zubereitung. Insbesondere ist die Packung/ Sachet flexibel. Die Zubereitungen sind ferner lagerstabil, insbesondere weisen sie keine unerwünschten Nebenwirkungen (Flatulationen) auf und sind physiologisch auch in höheren Mengen gut verträglich. Sie sind bei angemessener Süße vor allem kalorienarm (d.h. für Diabetiker geeignet) und lösen sich in der gewünschten Flüssigkeit im Wesentlichen rückstandsfrei.

Die erfindungsgemäßen Instant- Zubereitungen eignen sich daher zur raschen Herstellung von Kalt- und Heiß-Getränken wie Erfrischungs(Minz)tee, Heiße Ingwer/Zitrone, Fruchtcocktail, Mineralstofftee, u.ä. Dazu wird die in der Portionierpackung(Beutel) enthaltene Menge an Trockenprodukt in ein geeignetes Gefäß (Tasse/Glas) gegeben und sodann die gewünschte Flüssigkeit (Wasser) hinzugegeben. Es entsteht je nach Wirkstoffen/Zusatzstoffen eine sich geruchlich und farblich entsprechend darstellende flüssige sofort gebrauchsfertige Trinkzubereitung. Die Zubereitungen sind sowohl als Kaltgetränk (ggf. unter Rühren) als auch als Heißgetränk in Wasser sehr gut löslich, ohne dass bei ihrer Herstellung (s.o.) besondere Formen wie eine Schermatrixform bewirkt werden müsste.

### Beispiele

Die vorliegende Erfindung wird anhand der nachfolgenden Ausführungsbeispiele veranschaulicht, welche die vorliegende Erfindung jedoch nicht beschränken.

### Beispiel 1: Instant-Zubereitung "Heiße Zitrone"

Es wurde eine Mischung hergestellt aus folgenden Bestandteilen:

| Inhaltsstoffe | Anteil (%) g |
|---|---|
| Acelsulfam K | 0,252 |
| Aspartam | 0,252 |
| Cyclamat | 0,696 |
| Zitronensäure-Monohydrat | 17,300 |
| Ascorbinsäure | 5,750 |
| Zitronenaroma | 1,504 |
| Ingweraroma | 0,360 |
| Zinkglukonat | 0,70 |
| Riboflavon-5-Phosphat | 0,012 |
| Polydextrose | 73,174 |
| Summe | 100,000 |

### Beispiel 2: Heißgetränk "Salbei Honig"

| Inhaltsstoffe | Anteil (%), g |
|---|---|
| Acelsulfam K | 0,10 |
| Aspartam | 0,23 |
| Salbeiextrakt | 6,90 |
| Zitronensäure-Monohydrat | 1,51 |
| Ascorbinsäure | 6,00 |
| Salbeiaroma | 0,22 |
| Honigaroma | 0,12 |
| Zitronenaroma | 0,09 |
| Zinkglukonat | 0,70 |
| Siliciumdioxid | 0,21 |
| Pflanzliches Öl | 0,10 |
| Polydextrose | 83,82 |
| Summe | 100,00 |

### Beispiel 3: Heißgetränk "Acerola Salbei"

| Inhaltsstoffe | Anteil (%) g |
|---|---|
| Acesulfam-K | 0,16 |
| Aspartam | 0,40 |
| Zitronensäure-Monohydrat | 4,00 |
| Salbei-Extrakt | 1,00 |
| Acerola-Extrakt | 1,00 |
| Ascorbinsäure | 5,75 |
| Zinkgluconat | 0,70 |
| Salbeiaroma | 1,00 |
| Kirscharoma | 0,28 |
| Farbstoff | 6,40 |
| Polydextrose | 79,31 |
| Summe | 100,00 |

### Beispiel 4: Heißgetränk "Pelargonie Eucalyptus Minze"

| Inhaltsstoffe | Anteil (%) g |
|---|---|
| Acesulfam-K | 0,120 |
| Aspartam | 0,280 |
| Zitronensäure-Monohydrat | 3,200 |
| Pelargonium-Extrakt | 0,500 |
| Pfefferminz-Extrakt | 1,000 |
| Eucalyptus-Extrakt | 1,000 |
| Ascorbinsäure | 5,750 |
| Zinkgluconat | 0,700 |
| Pfefferminzaroma | 1,000 |
| Eucalyptusaroma | 0,400 |
| Polydextrose | 86,050 |
| Summe | 100,000 |

## Patentansprüche

1. Feste pulverförmige Instant-Zubereitung zur Herstellung von flüssigen Getränken, umfassend einen Trägerstoff, ein oder mehrere Wirkstoffe (Wirkstoffkomponente), ausgewählt aus Pflanzenextrakten, Pflanzenpulvern, Fruchtkonzentraten, Fruchtextrakten, Vitaminen, Mineralstoffen, Aminosäuren oder Kombinationen hiervon, weiterhin Zusatzstoffe, ausgewählt aus Geschmacksstoffen (= Aromastoffen), Säureregulatoren, Säuerungsmitteln, Füllstoffen, Farbstoffen, Trennmitteln, Süßungsmitteln oder Mischungen hiervon, **dadurch gekennzeichnet, dass** als Trägerstoff Polydextrose mit einer mittleren molaren Masse von 150 bis 10000 g/mol in einer Menge von mehr als 50 Gew.% enthalten ist.

2. Zubereitung nach Anspruch 1, **dadurch gekennzeichnet, dass** die Polydextrose eine mittlere molare Masse von 180 bis 5000 g/Mol aufweist.

3. Zubereitung nach Anspruch 1 oder 2, **dadurch gekennzeichnet, dass** sie 65 bis 95 Gew.%, insbesondere 75 bis 92 Gew.% Polydextrose, sowie weiterhin 0,1 bis 18 Gew.% eines oder mehrerer Wirkstoffe (Wirkstoffkomponente) und als Rest übliche Zusatzstoffe, ausgewählt aus Geschmacksstoffen, Säureregulatoren, Füllstoffen, Farbstoffen, Süßungsmitteln, Trennmitteln oder Mischungen hiervon aufweist.

4. Zubereitung nach einem der Ansprüche 1 bis 3, **dadurch gekennzeichnet, dass** sie 75-92 Gew. % Polydextrose, 0,1 bis 10 Gew.% eines oder mehrerer Wirkstoffe (Wirkstoffkomponente), ausgewählt aus Vitaminen, Mineralstoffen, Pflanzenextrakten, Fruchtextrakten, Pflanzenpulvern, Fruchtpulvern oder Mischungen hiervon und als Rest Zusatzstoffe aufweist, wobei der Gesamtrestfeuchtgehalt weniger als 5 Gew.% beträgt.

5. Zubereitung nach einem der Ansprüche 1 bis 4, **dadurch gekennzeichnet, dass** sie Polydextrose aufweist, die ausgewählt ist aus gereinigter reduzierter Polydextrose mit einem pH-Wert von 4,5 bis 6,5 oder aus gereinigter Polydextrose mit einem pH- Wert von 3,5 bis 5 oder Mischungen hiervon.

6. Zubereitung nach einem der Ansprüche 1 bis 5, **dadurch gekennzeichnet, dass** sie Polydextrose aufweist, die ausgewählt ist aus gereinigter reduzierter Polydextrose mit einem Reinheitsgrad von wenigstens 80%, insbesondere 90%, und einem Gehalt an reduziertem Zucker von max. 4 Gew.%, insbesondere max. 0,5 Gew.%.

7. Zubereitung nach einem der Ansprüche 1 bis 6, **dadurch gekennzeichnet, dass** ein oder mehrere Wirkstoffe, ausgewählt aus Vitamin C, Calcium, Magnesium, Zink in Form entsprechender Salze, ausgewählt aus Phosphat, Carbonat, Hydrogencarbonat, Gluconat, Extrakte und Pulver aus Pflanzen, Früchten, Säften, ausgewählt aus Melissenextrakt, Minzeextrakt, Hibiskusextrakt, Ananasextrakt, Mangoextrakt, Mate, Pelargoniumextrakt, Zitronenfruchtpulver, Salbeiextrakt, Ingwerwurzelpulver, Anis-, Fenchel, Lindenblüten-, Süßholzwurzelextrakt, Kakaobohnenextrakt, Eukalyptusextrakt, Pfefferminzextrakt, Orangensaftkonzentrat, Zitronenfruchtpulver, Hagebuttenextrakt, Acerolaextrakt, Bärentraubenextrakt, Weißdornfrüchtepulver, Ginkgoextrakt, Ginsengextrakt, Pelargoniumextrakt, Zitronengras, Guarana, Granatapfel, Cranberry, Birkenextrakt, Zitronengras, Guarana, enthalten sind.

8. Zubereitung nach einem der Ansprüche 1 bis 7, **dadurch gekennzeichnet, dass** sie im Wesentlichen frei ist von Zuckern und höher kalorischen Zuckeraustauschstoffen ausgewählt aus Glucose, Maltose, Saccharose (Kristallzucker), Mannose, Lactose, Xylose, Isomaltulose, Zuckeralkoholen aus der Gruppe, umfassend Erythrit (Erythritol), Mannit (Mannitol), Xylit (Xylitol), Sorbit (Sorbitol), Isomaltit bzw. Isomalt, Maltit (Maltitol), Lactit (Lactitol), Galactit (Galactitol) sowie deren Mischungen und/oder auch im Wesentlichen frei von Inulin ist.

9. Zubereitung nach einem der Ansprüche 1 bis 8, **dadurch gekennzeichnet, dass** Süßungsmittel, ausgewählt aus Honig, künstlichen Süßstoffen oder Mischungen hiervon, in einer Gesamtmenge von 0,1 bis 5 Gew. % enthalten sind.

10. Zubereitung gemäß einem der Ansprüche 1 bis 9, **dadurch gekennzeichnet, dass** sie in einer Menge von 3 bis 20 g in einer Portionspackung aus einem Beutel/Sachet aus Papier- oder Aluminiumfolie, einer Verbundfolie oder einer Mischung hieraus enthalten ist.

11. Verwendung von Polydextrose als Trägerstoff in einer Menge von mehr als 50 Gew.% zur Herstellung von festen, pulverförmigen, einen oder mehrere Wirkstoffe (Wirkstoffkomponente), ausgewählt aus Pflanzenextrakten, Pflanzenpulvern, Fruchtkonzentraten, Fruchtextrakten, Vitaminen, Mineralstoffen, Aminosäuren, enthaltenden Instantzubereitungen zur Herstellung von Getränken.

12. Verwendung gemäß Anspruch 11 , **dadurch gekennzeichnet, dass** die Polydextrose, ausgewählt ist Polydextrose mit einer mittleren molaren Masse von ca. 150 bis 10000 g/Mol, insbesondere 180 bis 5000 g/Mol.

13. Verwendung gemäß einem der Ansprüche 11 oder 12, **dadurch gekennzeichnet, dass** die Instantzubereitungen in einer Portionspackung aus einem Beutel/Sachet aus Papier- oder Aluminiumfolie, einer Verbundfolie oder einer Mischung hieraus enthalten sind.
